# EUROPÄISCHE PATENTANMELDUNG

(11) **EP 2 251 051 A2**
(43) Veröffentlichungstag der Anmeldung: **17.11.2010**
(21) Anmeldenummer: 10156627.1
(22) Anmeldetag: 16.03.2010
(51) Int. Cl.: A61L 29/08

(54) **Katheter mit einschichtigem Innenschaft**

(30) Priorität: 14.05.2009 DE 102009003114
(71) Anmelder: Biotronik VI Patent AG, 6341 Baar (CH)
(72) Erfinder: Schwitzer, Alwin, 8180, Bülach (CH); Surber, Bettina, CH-8547 Gachnang (CH)
(74) Vertreter: Lindner-Vogt, Karin L.

(57) **Zusammenfassung**

Die Erfindung bezieht sich auf einen Katheter mit cinschichtigcm Inncnschlauch, wobei der einschichtige Innenschlauch mit einer Innenfläche ein Lumen und mit einer Außenfläche eine äußere Oberfläche eines Katheterrohres bildet, wobei im Lumen des Innenschlauchs ein oder mehrere Führungsdrähte anordenbar sind, **dadurch gekennzeichnet, dass** der einschichtige Innenschlauch aus einem Material besteht, umfassend: a) 96 bis 99,9 Gew.% eines Polymers, eines Copolymers und/oder einer Mischung aus mehreren Polymeren und/oder Copolymeren; b) 0 bis 1 Gew.% Talk; c) 0 bis 1 Gew.% Calziumcarbonat; d) 0 bis 1 Gew.% Siliziumdioxid; e) 0 bis 1 Gew.% Wachse; wobei die Summe der Gew.% der Komponenten b) bis e) mindestens 0,1 Gew.% und nicht mehr als 4 Gew.% beträgt und wobei sich alle Angaben in Gew.% auf das Gesamtgewicht des einschichtigen Innenschlauchs beziehen.

## Beschreibung

### Technisches Gebiet

Die Erfindung betrifft das Gebiet der Katheter, insbesondere von Kathetern zur Anwendung in der Angioplastie, sowie das Gebiet der Herstellverfahren für Bestandteile solcher Katheter.

### Technologischer Hintergrund und Stand der Technik

Katheter, insbesondere drahtgeführte Katheter, haben eine breite Verwendung bei vorwiegend medizinischen Anwendungen und Eingriffen gefunden. Insbesondere in der Angioplastie und bei der Implantation von Stents werden drahtgeführte Katheter eingesetzt.

Es sind verschiedenste drahtgeführte Kathetertypen bekannt, so beispielsweise interventionelle Katheter, Ballonkatheter und Katheter zur Applikation von selbstexpandierenden Stents. Allen drahtgeführten Kathetern ist gemein, dass sie einen Schlauch aufweisen, den sogenannten Innenschlauch, in dessen Lumen die Führungsdrähte angeordnet sind. Durch geeignete Manipulation der Führungsdrähte lässt sich der Katheter gerichtet bewegen und beispielsweise im menschlichen Gefäßsystem an eine gewünschte Stelle leiten. Dabei entstehen Reibungskräfte zwischen der Innenoberfläche des Innenschlauchs und den Führungsdrähten. Diese Reibungskräfte können die Funktion und die Sicherheit des Katheters beeinträchtigen.

Es sind mehrere Ansätze bekannt, diese Reibung zwischen Führungsdraht und Innenschlauch zu vermindern.

So wird z. B. in DE 696 36 202 eine Katheteranordnung beschrieben, bei der ein mehrschichtiger Innenschlauch verwendet wird. Die innerste Schicht des Innenschlauchs weist dabei eine Oberfläche mit geringem Reibungskoeffizienten auf und ist mit weiteren Schichten verklebt, die für die notwendige Stabilität des Schlauchs sorgen.

Ein anderer Ansatz ist, die Innenfläche des Innenschlauchs mit einem Gleitmittel zu beschichten, welches den Reibungskoeffizienten der Schlauchoberfläche herabsetzt.

Den bekannten Lösungen ist gemein, dass sie aufwendige Maßnahmen verlangen, kostenintensiv sind und mehrere Schritte oder Manipulationen bei der Herstellung des Innenschlauches oder des Katheters voraussetzen.

Aufgabe der vorliegenden Erfindung ist es einen oder mehrere Nachteile des Standes der Technik zu mindern oder zu verhindern. Insbesondere sollen Katheter bereitgestellt werden, die eine reduzierte Reibung zwischen Innenschlauch und Führungsdraht aufweisen und einfach, kostengünstig und/oder in weniger Schritten herstellbar sind.

### Erfindungsgemäße Lösung

Die Aufgabe wird gelöst durch Bereitstellung eines Katheters mit einschichtigem Innenschlauch, wobei der einschichtige Innenschlauch mit einer Innenfläche ein Lumen und mit einer Außenfläche eine äußere Oberfläche eines Katheterrohres bildet, wobei im Lumen des Innenschlauchs ein oder mehrere Führungsdrähte anordenbar oder angeordnet sind, dadurch gekennzeichnet, dass der einschichtige Innenschlauch aus einem Material besteht, umfassend
a) **96 bis 99,9** Gew.% eines Polymers, eines Copolymers und/oder einer Mischung aus mehreren Polymeren und/oder Copolymeren;
b) 0 bis 1 Gew.% Talk;
c) 0 bis 1 Gew.% Calziumcarbonat;
d) 0 bis 1 Gew.% Siliziumdioxid;
e) 0 bis 1 Gew.% Wachs oder Wachsgemische;
wobei die Summe der Gew.% der Komponenten b) bis e) mindestens 0,1 Gew.% und nicht mehr als 4 Gew.% beträgt und wobei sich alle Angaben in Gew.% auf das Gesamtgewicht des einschichtigen Innenschlauchs beziehen.

Durch Zugabe einer oder mehrerer Komponenten b) bis e) zu dem hauptsächlichen Polymer des Innenschlauchs bei oder vor der Ausformung des Innenschlauchs, wird eine Innenschlauchoberfläche erhalten, die zu einer geringeren Reibung zwischen Führungsdraht und Schlauchoberfläche führt. Der Effekt beruht darauf, dass die Innenschlauchoberfläche durch die Komponenten leicht aufgeraut wird, so dass sich die Kontaktfläche zwischen Draht und Innenschlauch verringert. Da nun weniger Fläche für den Kontakt zur Verfügung steht, nimmt die Reibung insgesamt ab. Diese Reduktion der Reibung reicht bereits aus, um einen problemlosen und sicheren Betrieb des Katheters zu gewährleisten ohne dass der Einsatz von zusätzlichen Gleit oder Schmiermitteln erforderlich ist. Da die Komponenten dem Grundpolymer des Innenschlauchs bei oder vor der Extrusion zugesetzt werden können, ist es möglich einen einschichtigen Innenschlauch in wenigen Schritten herzustellen, der die gewünschten Eigenschaften aufweist. Somit entfallen die aufwendigen Verfahren und Schritte zur Herstellung von mehrschichtigen Innenschläuchen. Es ist nun auch nicht mehr notwendig, nach der Herstellung eines Innenschlauchs, dessen Oberfläche durch Einsatz von zusätzlichen Gleitmitteln gebrauchsfertig zu machen.

Grundsätzlich umfasst der erfindungsgemäße Katheter alle drahtgeführten Kathetertypen, wie beispielsweise interventionelle Katheter, Katheter zur Stentapplikation, Ballonkatheter oder dergleichen, und ist nicht auf einen Kathetertyp beschränkt. Wesentlich ist, dass der Katheter einen erfindungsgemäßen einschichtigen Innenschlauch aufweist.

Der einschichtige Innenschlauch des Katheters bildet mit seiner Innenfläche ein Lumen, in dem ein oder mehrere Führungsdrähte des Katheters anordenbar oder angeordnet sind. Nach außen bildet die Oberfläche des einschichtigen Innenschlauchs eine äußere Oberfläche eines Katheterrohres. Der überwiegende Teil der äußeren Oberfläche des einschichtigen Innenschlauchs steht nicht direkt mit weiteren Schichten des Katheters in Kontakt, sondern der einschichtige Innenschlauch bildet ein eigenständiges Katheterrohr, welches funktional mit anderen Katheterbestandteilen, z. B. auch anderen Katheterrohren, in Verbindung stehen kann.

Der Innenschlauch weist einen einschichtigen Aufbau auf. Die Materialien, die zur Herstellung des Innenschlauchs verwendet werden liegen dabei über den Querschnitt der Innenschlauchfläche zufällig verteilt, insbesondere gleich verteilt vor. Es sind keine Bereiche im Innenschlauchs vorhanden, die sich in der Materialzusammensetzung wesentlich von anderen Bereichen des Innenschlauchs unterscheiden.

Der einschichtige Innenschlauch besteht aus einem Material, umfassend
a) **96 bis 99,9** Gew.% eines Polymers, eines Copolymers und/oder einer Mischung aus mehreren Polymeren und/oder Copolymeren, bevorzugt eines Polyamids, Polyamidcopolymeren oder polyamid-haltigen Copolymeren, insbesondere Polyamid-12, Polyamid-12/6 und/oder Polyether-Blockamide (z. B. PEBAX®) bzw. Mischungen davon;
b) 0 bis 1 Gew.% Talk;
c) 0 bis 1 Gew.% Calciumcarbonat;
d) 0 bis 1 Gew.% Siliziumdioxid;
e) 0 bis 1 Gew.% Wachs oder Wachsgemische;
wobei die Summe der Gew.% der Komponenten b) bis e) mindestens 0,1 Gew.% und nicht mehr als 4 Gew.% beträgt und wobei sich alle Angaben in Gew.% auf das Gesamtgewicht des einschichtigen Innenschlauchs beziehen.

Der einschichtige Innenschlauch besteht aus einem Material enthaltend ein Polymer, ein Copolymer und/oder einer Mischung aus mehreren Polymeren und/oder Copolymeren. Grundsätzlich können hier Polymere oder Copolymere eingesetzt werden, die die nötige Stabilität und Biegsamkeit für den Einsatz in einem Katheter aufweisen und mit den Komponenten b) bis e) in den angegebenen Konzentrationen mischbar sind. Dem Fachmann sind solche Polymere und Copolymere bzw. Mischungen daraus aus dem Stand der Technik bekannt. Die Verträglichkeit bzw. Mischbarkeit mit den Komponenten b) bis e) sowie andere Eigenschaften des Polymers kann der Fachmann in einfachen Routineversuchen ohne Schwierigkeiten bestimmen. Bevorzugt werden Polymere und/oder Copolymere verwendet, die mindestens ein sich wiederholendes Monomer mit mindestens einer Amidgruppe aufweisen. Insbesondere können Polyamide, Polyamidcopolymere oder polyamid-haltige Copolymere eingesetzt werden. Dabei sind Polyamide mit mindestens 6 C-Atomen pro Monomer bevorzugt, besonders bevorzugt mit mehr als 10, ganz besonders bevorzugt mit 12 C-Atomen. Insbesondere können Polyamid-12 oder Polyamid-12-Copolymere, wie z. B. Polyamid-12/6 eingesetzt werden. Auch Polyether-Blockamide (z. B. solche unter dem Markennamen PEBAX®) können verwendet werden. Des Weiteren können bevorzugt Polymere oder Copolymere der Gruppe der Polyester, Copolyester und/oder Polyester-Elastomere eingesetzt werden.

Talk, auch Magnesiumsilikathydrat, ist ein Mineral mit der chemischen Zusammensetzung Mg₃Si₄O₁₀(OH)₂. Bevorzugt wird Talk mit der CAS-Nr. 14807-96-6 verwendet.

Grundsätzlich kann jedes Calciumcarbonat eingesetzt werden, bevorzugt wird precipitated Calciumcarbonat mit der CAS-Nr. 72608-12-9 eingesetzt.

Grundsätzlich kann jedes Siliziumdioxid eingesetzt werden, bevorzugt wird precipitated Siliziumdioxid mit der CAS-Nr. 7631-86-9 eingesetzt.

Bevorzugt werden Stearamid-Wachse z. B.: N,N'_Ethylen-bis-stearamid (CAS-Nr. 110-30-5) und/oder Montanwachse (CAS-Nr. 8002-53-7), speziell Kalzium-Montanate (CAS-Nr. 68308-22-5) und/oder Glyceryl-Montanate (CAS-Nr. 68476-38-0) eingesetzt, entweder einzeln oder als Wachsgemische.

Die Komponenten b) bis e) liegen in einer Konzentration von jeweils 0 bis 1 Gew.% vor, bevorzugt von jeweils 0 bis 0,5 Gew.%, wobei die Summe der Gew.% der Komponenten b) bis e) mindestens 0,1 Gew.%, bevorzugt mindestens 0,5 Gew.% und nicht mehr als 4 Gew.%, bevorzugt nicht mehr als 2 Gew.% beträgt. Alle Angaben in Gew.% beziehen sich auf das Gesamtgewicht des Materials des einschichtigen Innenschlauchs. Dabei ist es nicht notwendig, dass alle Komponenten b) bis e) vorhanden sind. Das Material des einschichtigen Innenschlauchs kann eine, zwei, drei und/oder vier der Komponenten b) bis e) ggf. in unterschiedlichen Anteilen enthalten.

Der erfindungsgemäße Katheter kann weitere Bestandteile umfassen. Beispielsweise kann der Katheter einen dilatierbaren Ballon aufweisen.

Der erfindungsgemäße Katheter kann zusätzlich zum einschichtigen Innenschlauch auch einen ein- oder mehrschichtigen Außenschlauch aufweisen. Dabei kann der Außenschlauch den Innenschlauch mindestens teilweise oder ganz umgeben. Sowohl beim Außenschlauch als auch beim einschichtigen Innenschlauch handelt es sich um eigenständige unabhängige Schläuche, die über nicht mehr als 50% der Außenoberfläche des Innenschlauchs direkt miteinander in Kontakt stehen. Es kann zwischen Innenoberfläche des Außenschlauchs und Außenoberfläche des Innenschlauchs ein Lumen ausgebildet sein. Das Lumen kann beispielsweise dazu genutzt werden, Verbindungen oder Stoffe vom proximalen Ende des Katheters in Richtung des distalen Endes des Katheters zu befördern. Ist der erfindungsgemäße Katheter ein Ballonkatheter, so kann das Lumen zwischen Außen- und Innenschlauch dazu dienen, den dilatierbaren Ballon mit einem Fluid zu inflatieren.

Die vorliegende Erfindung bezieht sich auch auf ein Verfahren zur Herstellung eines einschichtigen Innenschlauchs für einen erfindungsgemäßen Katheter, dadurch gekennzeichnet, dass
a) mindestens zwei Vorstufen verwendet werden, wobei die erste Vorstufe ein Polymer oder Copolymer, bevorzugt ein Polyamid, Polyamidcopolymer oder polyamid-haltiges Copolymer umfasst und die zweite Vorstufe mindestens eine der Komponenten Talk, Calciumcarbonat, Siliziumdioxid und Wachs oder Wachsgemische enthält;
b) beide Vorstufen miteinander gemischt werden und
c) das erhaltene Materialgemisch zur Ausbildung des Innenschlauchs eingesetzt wird.

Die Mischung der beiden Vorstufen miteinander und das Verfahren zur Ausbildung des Innenschlauchs können auch parallel in einem gemeinsamen Schritt erfolgen Ähnlich wie beim Einfärben (Colorieren) von Thermoplasten, können die beiden Vorstufen erst beim Herstellen des Innenschlauchs miteinander gemischt und aufgeschmolzen werden.

Dem Fachmann sind Verfahren zur Ausbildung eines Innenschlauchs ausgehend von einem Polymer-enthaltenden Material bekannt. Bevorzugt wird der erfindungsgemäße einschichtige Innenschlauch mit einem Extrusionsverfahren hergestellt. Andere geeignete Verfahren sind beispielsweise Spritzgussverfahren und Blasfolien-Extrusion.

Die erste Vorstufe enthält ein Polymer, ein Copolymer und/oder einer Mischung aus mehreren Polymeren und/oder Copolymeren. Grundsätzlich können im erfindungsgemäßen Verfahren Polymere oder Copolymere eingesetzt werden, die die nötige Stabilität und Biegsamkeit für den Einsatz in einem Katheter aufweisen und mit den Komponenten b) bis e) in den angegebenen Konzentrationen mischbar sind. Dem Fachmann sind solche Polymere und Copolymere bzw. Mischungen daraus aus dem Stand der Technik bekannt. Die Verträglichkeit bzw. Mischbarkeit mit den Komponenten b) bis e) sowie andere Eigenschaften des Polymers kann der Fachmann in einfachen Routineversuchen ohne Schwierigkeiten bestimmen. Bevorzugt werden Polymere und/oder Copolymere verwendet, die mindestens ein sich wiederholendes Monomer mit mindestens einer Amidgruppe aufweisen. Insbesondere können Polyamide, Polyamidcopolymere oder polyamid-haltige Copolymere eingesetzt werden. Dabei sind Polyamide mit mindestens 6 C-Atomen pro Monomer bevorzugt, besonders bevorzugt mit mehr als 10, ganz besonders bevorzugt mit 12 C-Atomen. Insbesondere können Polyamid-12 oder Polyamid-12-Copolymere, wie z. B. Polyamid-12/6 eingesetzt werden. Auch Polyether-Blockamide (z. B. solche unter dem Markennamen PEBAX®) können verwendet werden. Des Weiteren können bevorzugt Polymere oder Copolymere der Gruppe der Polyester, Copolyester und/oder Polyester-Elastomere eingesetzt werden.

Die vorliegende Erfindung bezieht sich auch auf einen Katheter umfassend einen einschichtigen Innenschlauch, hergestellt nach einem der oben genannten erfindungsgemäßen Verfahren. Dabei können die erfindungsgemäßen Katheter dadurch hergestellt werden, dass ein herkömmliches Herstellverfahren genutzt wird, wobei der bisherige Innenschlauch durch einen, nach einem der oben genannten erfindungsgemäßen Verfahren hergestellten, einschichtigen Innenschlauch ersetzt wird.

Figuren:
- FIG. 1: zeigt eine beispielhafte Ausführungsform einer erfindungsgemäßen Appli- kationsvorrichtung als Ballonkatheter.
- FIG. 2: zeigt einen Querschnitt durch die beispielhafte Ausführungsform aus FIG. 1.

Die Erfindung wird nachfolgend anhand der Ausführungsbeispiele näher erläutert.

### BEISPIEL 1:

### Herstellung eines einschichtigen Innenschlauchs

### Methode 1

Die Granulate der Komponenten a) und b) werden folgendermaßen gemischt. Zu 97 Gew.% Polyamid 12, Grilamid L25, EMS-GRIVORY werden 3 Gew.% des Masterbatches Grilon C MB 7361 FS, EMS-GRIVORY, enthaltend die Komponenten b) bis e) in folgenden Konzentrationen:
b): 0-10% Talk
c): 0-10% Calziumcarbonat
d): 0-10% Siliziumdioxid
e): 0-10% Wachsgemische in
f): 60-99% eines PA612 Copolyamids
gegeben, homogenisiert und getrocknet. Die Mischung wird auf einer handelsüblichen Profil-Extrusionsanlage (L/D 24-28), bevorzugt auf einer Profil-Extrusionsanlage mit D <= 20 mm mit einer geeigneten Düse/Dorn Geometrie, evtl. mit Unterstützung von Stützluft, zum Innenschlauch extrudiert, auf die gewünschten Durchmesser kalibriert, bevorzugt über ein Vakuum-Kalibrier-Becken, und in einem Wasserbad abgekühlt. Die Dimensionen und/oder Wandstärken werden online kontrolliert. Zusätzlich können die extrudierten Innenschläuche online einer visuellen Inspektion auf Fehlstellen unterzogen werden. Die für gut befundenen Abschnitte, des auf diese Weise hergestellten endlosen Innenschlauchs, werden online herausgeschnitten und staubfrei verpackt.

### Methode 2

Die Granulate der Komponenten a) und b) werden folgendermaßen gemischt. Zu 96 Gew.% Polyamid 12, Grilamid L25, EMS-GRIVORY werden 4 Gew.% des Masterbatches Grilon C MB 8361 FS, EMS-GRIVORY enthaltend die Komponenten b) bis e) in folgenden Konzentrationen:
b): 0-10% Talk
c): 0-10% Calziumcarbonat
d): 0-10% Siliziumdioxid
e): 0-10% Wachsgemische in
f): 60-99% eines PA612 Copolyamids
gegeben, homogenisiert und getrocknet. Die Mischung wird auf einer handelsüblichen Profil-Extrusionsanlage (L/D 24-28), bevorzugt auf einer Profil-Extrusionsanlage mit D <= 20 mm mit einer geeigneten Düse/Dorn Geometrie auf einen Draht, zur Einstellung des Innendurchmessers extrudiert, auf den gewünschten Außendurchmesser kalibriert, bevorzugt über ein Vakuum-Kalibrier-Becken, und in einem Wasserbad abgekühlt. Die Dimensionen und/oder Wandstärken werden online kontrolliert. Zusätzlich können die Extrudate online einer visuellen Inspektion auf Fehlstellen unterzogen werden. Die für gut befundenen Abschnitte, des auf diese Weise hergestellten endlosen Extrudats, werden online herausgeschnitten. In einem letzten Schritt werden die Drahtabschnitte aus den Extrudatabschnitten herausgezogen um die Innenschläuche zu erhalten. Diese werden staubfrei verpackt.

### BEISPIEL 2:

### Beschreibung einer erfindungsgemäßen Ausführungsform als Ballonkatheter

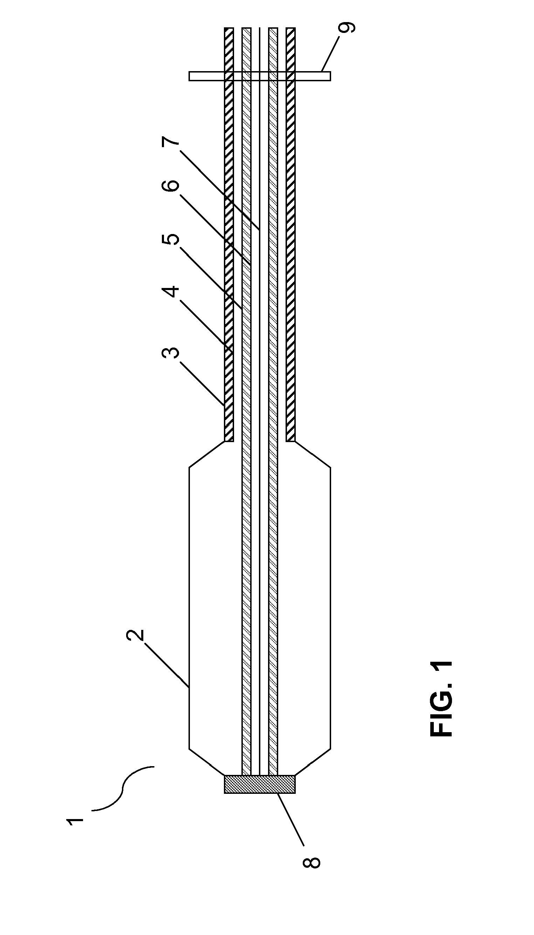

In FIG. 1 ist ein Ballonkatheter 1 dargestellt mit einem einschichtigen Innenschlauch 5, an dem ein distales Ende eines expandierbaren Ballons 2 befestigt ist, der in einem nichtexpandierten, deflatierten Zustand an einer Außenoberfläche des Innenschlauchs 5 zumindest teilweise anliegt. Der Ballonkatheter 1 kann auf der Außenseite des Ballons 2 einen Stent aufweisen, der nach Einführen in ein Gefäß durch Inflation des Ballons 2 mit einem Fluid expandiert und so am vorgesehenen Ort an eine Gefäßwand gedrückt wird.

Der Ballonkatheter 1 weist neben einem einschichtigen Innenschlauch 5 und dem Ballon 2 auch einen Außenschlauch 3 auf, der wenigstens bis zu einem proximalen Ende des Ballons 2 reicht und mit diesem fluiddicht verbunden ist. Zwischen Innenschlauch 5 und Außenschlauch 3 des Katheters 1 ist üblicherweise eine in Längsrichtung des Katheters 1 von seinem proximalen Ende bis ins Innere des Ballons 2 reichende Fluidleitung 4 vorgesehen, die sich beispielsweise daraus ergibt, dass ein Lumen geschaffen ist zwischen Außenschlauch 3 und Innenschlauch 5 in dem der Außenschlauch 3 einen Innendurchmesser besitzt, der größer ist, als ein Außendurchmesser des Innenschlauchs 5.

Im Inneren des einschichtigen Innenschlauchs 5 ist ein vom Innenschlauch 5 eingeschlossener, sich in Längsrichtung des Innenschlauchs 5 erstreckender Hohlraum als Lumen 6 vorgesehen. Dieses Lumen 6 dient der Aufnahme eines Führungsdrahtes 7. Katheter 1 und Führungsdraht 7 sind dann beispielsweise so ausgebildet, dass der Führungsdraht 7 an der distalen Spitze 8 des Katheters 1 austreten kann und von seinem proximalen Ende her zu steuern ist. Der Führungsdraht 7 wird zum Beispiel mit Hilfe von Steuermitteln so ausgelenkt, dass er auch in abzweigende Blutgefässe leicht einzuführen ist. Aufgrund der Eigenschaften des einschichtigen Innenschlauchs 5, ist die Reibung zwischen Innenoberfläche des einschichtigen Innenschlauchs 5 und Führungsdraht 7 derart verringert, dass ein problemloser und sicherer Betrieb des Katheters 1 gewährleistet ist. Der Ballonkatheter 1 kann entlang des Führungsdrahtes 7 nachgeschoben werden.

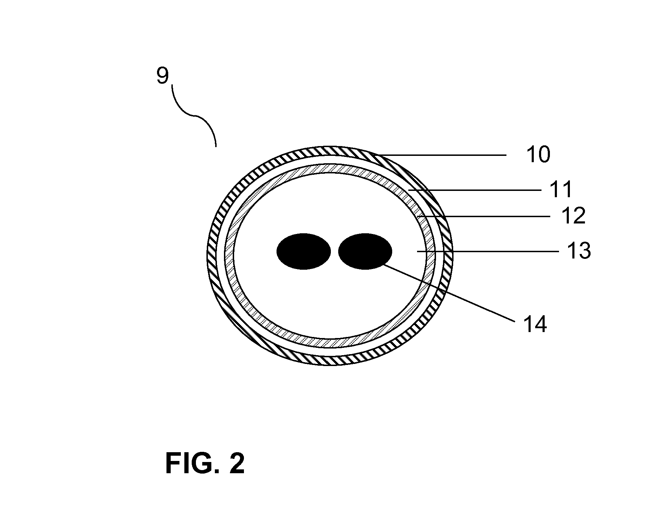

An seinem distalen Ende weist der Ballonkatheter 1 den bereits erwähnten, expandierbaren Ballon 2 auf. Während des Einführens des Ballonkatheters 1 ist der Ballon 2 komprimiert und liegt eng am einschichtigen Innenschlauch 5 des Katheters 1 an. Durch Inflatieren des Ballons 2 mit einem Fluid kann dieser expandiert werden. Dieses Expandieren des Ballons 2 geschieht, sobald der Ballon 2 bis zu der bestimmungsgemäßen Position geführt ist. Schließlich ist in FIG. 1 die Ebene eines Querschnitts 9 durch den Ballonkatheter 1 gezeigt, wobei Details des Querschnitts 9 in FIG. 2 dargestellt sind.

In FIG. 2 ist ein Querschnitt 9 durch den Ballonkatheter 1 aus FIG. 1 mit seiner Schlauchanordnung gezeigt. Dabei ist zu sehen, wie der Führungsdraht 14 im Lumen 13 des einschichtigen Innenschlauchs 12 angeordnet ist, während der einschichtige Innenschlauch 12 von einem Außenschlauch 10 umgeben ist. Zwischen einschichtigem Innenschlauch 12 und Außenschlauch 10 besteht eine ringförmige Fluidleitung 11, die den einschichtigen Innenschlauch 12 umgibt und durch welche der Ballon mit Fluid inflatiert werden kann. Aus FIG. 2 ergibt sich, dass bei dem erfindungsgemäßen Katheter der einschichtige Innenschlauch 12 als eigenständiger Schlauch ausgebildet ist, der im Wesentlichen unabhängig ist von einem ggf. vorhandenen Außenschlauch 10.

## Patentansprüche

1. Katheter mit einschichtigem Innenschlauch, wobei der einschichtige Innenschlauch mit einer Innenfläche ein Lumen und mit einer Außenfläche eine äußere Oberfläche eines Katheterrohres bildet, wobei im Lumen des Innenschlauchs ein oder mehrere Führungsdrähte anordenbar sind,
**dadurch gekennzeichnet, dass**
der einschichtige Innenschlauch aus einem Material besteht, umfassend
a) **96 bis 99,9** Gew.% eines Polymers, eines Copolymers und/oder einer Mischung aus mehreren Polymeren und/oder Copolymeren;
b) 0 bis 1 Gew.% Talk;
c) 0 bis 1 Gew.% Calziumcarbonat;
d) 0 bis 1 Gew.% Siliziumdioxid;
e) 0 bis 1 Gew.% Wachse;
wobei die Summe der Gew.% der Komponenten b) bis e) mindestens 0,1 Gew.% und nicht mehr als 4 Gew.% beträgt und wobei sich alle Angaben in Gew.% auf das Gesamtgewicht des einschichtigen Innenschlauchs beziehen.

2. Katheter nach Anspruch 1, **dadurch gekennzeichnet, dass** der Katheter einen Außenschlauch aufweist, der den einschichtigen Innenschlauch mindestens teilweise umgibt.

3. Katheter nach Anspruch 1, **dadurch gekennzeichnet, dass** zwischen Außen- und Innenschlauch ein Lumen ausgebildet ist.

4. Katheter nach einem der vorhergehenden Ansprüche, **dadurch gekennzeichnet, dass** der Katheter einen dilatierbaren Ballon aufweist.

5. Katheter nach einem der vorhergehenden Ansprüche, **dadurch gekennzeichnet, dass** der einschichtige Innenschlauch aus einem Material besteht, welches mindestens zwei der Komponenten b) bis e) aufweist, bevorzugt drei, besonders bevorzugt alle vier Komponenten.

6. Katheter nach einem der vorhergehenden Ansprüche, **dadurch gekennzeichnet, dass** der einschichtige Innenschlauch aus einem Material besteht, umfassend
a) **96 bis 99,9** Gew.% eines Polymers, eines Copolymers und/oder einer Mischung aus mehreren Polymeren und/oder Copolymeren;
b) 0 bis 0,5 Gew.% Talk;
c) 0 bis 0,5 Gew.% Calziumcarbonat;
d) 0 bis 0,5 Gew.% Siliziumdioxid;
e) 0 bis 0,5 Gew.% Wachse;
wobei die Summe der Gew.% der Komponenten b) bis e) mindestens 0,1 Gew.% und nicht mehr als 2 Gew.% beträgt und wobei sich alle Angaben in Gew.% auf das Gesamtgewicht des einschichtigen Innenschlauchs beziehen.

7. Katheter nach einem der vorhergehenden Ansprüche, **dadurch gekennzeichnet, dass** die Summe der Gew.% der Komponenten b) bis e) mindestens 0,5 Gew.% und nicht mehr als 2 Gew.% beträgt und wobei sich alle Angaben in Gew.% auf das Gesamtgewicht des einschichtigen Innenschlauchs beziehen.

8. Verfahren zur Herstellung eines einschichtigen Innenschlauchs für einen Katheter nach einem der Ansprüche 1 bis 7, **dadurch gekennzeichnet, dass**
a) mindestens zwei Vorstufen verwendet werden, wobei die erste Vorstufe ein Polymer oder Copolymer, bevorzugt ein Polyamid, Polyamidcopolymer oder polyamid-haltiges Copolymer und die zweite Vorstufe mindestens eine der Komponenten b) bis e) enthält;
b) beide Vorstufen miteinander gemischt werden und
c) das erhaltene Materialgemisch zur Ausbildung des Innenschlauchs eingesetzt wird.

9. Verfahren zur Herstellung eines einschichtigen Innenschlauchs für einen Katheter nach einem der Ansprüche 1 bis 7, **dadurch gekennzeichnet, dass** der einschichtige Innenschlauch mit Hilfe eines Extrusionsverfahrens ausgebildet wird.

10. Katheter umfassend einen einschichtigen Innenschlauch hergestellt nach einem Verfahren gemäß Ansprüchen 8 oder 9.
